# EUROPEAN PATENT APPLICATION

(11) **EP 4 481 033 A1**
(43) Date of publication of application: **25.12.2024**
(21) Application number: 23771039.7
(22) Date of filing: 10.03.2023
(51) Int. Cl.: C12N 1/20, C12N 9/00, C12N 15/52, C12P 13/04

(54) **L-AMINO ACID-PRODUCING MICROORGANISM HAVING WEAKENED CARBAMOYL PHOSPHATE SYNTHASE ACTIVITY, AND L-AMINO ACID PRODUCTION METHOD USING SAME**

(30) Priority: 18.03.2022 KR 20220034098
(71) Applicant: CJ CheilJedang Corporation, Seoul 04560 (KR)
(72) Inventor: PARK, Jinsub, Seoul 04560 (KR); BYUN, Hyo Jeong, Seoul 04560 (KR); LEE, Ji Yeon, Seoul 04560 (KR); HAN, Seunghee, Seoul 04560 (KR)
(74) Representative: Meissner Bolte Partnerschaft mbB
(86) International application number: PCT/KR2023/003302
(87) International publication number: WO 2023/177160

(57) **Abstract**

Provided are a microorganism producing L-amino acids, in which activity of carbamoyl phosphate synthetase is weakened, and a method of producing L-amino acids using the same.

## Description

### [Technical Field]

The present disclosure relates to a microorganism producing L-amino acids, in which activity of carbamoyl phosphate synthetase is weakened, and a method of producing L-amino acids using the same.

### [Background Art]

In a process of producing L-amino acids using microorganisms, ATP is an important substance that is used as a cofactor for biosynthetic enzymes or used in cell growth and various metabolic processes for active maintenance.

Carbamoyl phosphate is an intermediate metabolite used in the pyrimidine biosynthetic pathway and the arginine biosynthetic pathway. Carbamoyl phosphate synthetase is an enzyme that converts bicarbonate to carbamoyl phosphate, and utilizes two molecules of ATP and one molecule of glutamine as a nitrogen donor when producing carbamoyl phosphate. Glutamine is produced by glutamine synthetase from glutamate as a substrate, and in this process, one molecule of ATP is consumed.

In this regard, it has been reported that when a variant carbamoyl phosphate synthetase that desensitizes feedback inhibition and maintains high activity is introduced into E. *coli,* the production of L-arginine, citrulline, and pyrimidine derivatives increases (Patent Document 1).

However, it has been reported that excessive intracellular formation of carbamoyl phosphate causes cytotoxicity (Non-Patent Document 1). Carbamoyl phosphate is a very unstable substance, and cyanate, which is naturally converted from carbamoyl phosphate, may cause cytotoxicity.

Accordingly, if the activity of carbamoyl phosphate synthetase in microorganisms is controlled to maintain an appropriate intracellular level of carbamoyl phosphate, to prevent unnecessary waste of ATP, and to control energy balance, it is possible to improve L-amino acid production.

### [Prior Art Documents]

### [Patent Documents]

(Patent Document 1) JP 2003-093083 A

### [Non-Patent Documents]

(Non-Patent Document 1) Charlier et al., Amino Acids. 2018; 50(12): 1647-1661.

### [Disclosure]

### [Technical Problem]

The present inventors have developed a microorganism of the genus *Corynebacterium* having L-amino acid-producing ability, in which activity of carbamoyl phosphate synthetase is weakened, a method of producing L-amino acids using the microorganism, a composition for producing L-arginine, the composition comprising the microorganism, and use of the microorganism in producing L-arginine, thereby completing the present disclosure.

### [Technical Solution]

An object of the present disclosure is to provide a microorganism of the genus *Corynebacterium* having L-amino acid-producing ability, in which activity of carbamoyl phosphate synthetase is weakened.

Another object of the present disclosure is to provide a method of producing L-amino acids, the method comprising the step of culturing, in a medium, a microorganism of the genus *Corynebacterium* having L-amino acid-producing ability, in which activity of carbamoyl phosphate synthetase is weakened.

Still another object of the present disclosure is to provide a composition for producing L-amino acids, the composition comprising the microorganism of the genus *Corynebacterium* of the present disclosure; a medium in which the microorganism is cultured; or a combination thereof.

Still another object of the present disclosure is to provide a method of preparing a microorganism of the genus *Corynebacterium* having L-amino acid-producing ability, the method comprising the step of weakening carbamoyl phosphate synthetase.

Still another object of the present disclosure is to provide use of a microorganism of the genus *Corynebacterium,* in which activity of carbamoyl phosphate synthetase is weakened, in producing L-amino acids.

### [Advantageous Effects]

A microorganism of the present disclosure may have increased L-amino acid-producing ability by weakening the activity of carbamoyl phosphate synthetase, as compared to existing unmodified microorganisms.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a microorganism of the genus *Corynebacterium* having L-amino acid-producing ability, in which activity of carbamoyl phosphate synthetase is weakened.

As used herein, the term "carbamoyl phosphate synthetase" refers to an enzyme that converts bicarbonate to carbamoyl phosphate.

The carbamoyl phosphate synthetase may consist of a carbamoyl phosphate synthetase small subunit (CarA) and a carbamoyl phosphate synthetase large subunit (CarB), and an amino acid sequence of CarA or CarB may be obtained from NCBI's Genbank which is a known database, etc.

For example, CarA or CarB of the present disclosure may be derived from a microorganism. The microorganism may be derived from, specifically, a microorganism of the genus Corynebacterium, more specifically, *Corynebacterium glutamicum, Corynebacterium deserti, Corynebacterium crudilactis, Corynebacterium efficiens, Corynebacterium callunae,* etc., but is not limited thereto.

For another example, an amino acid sequence of CarA of the present disclosure may be ANU33813.1 derived from *Corynebacterium glutamicum* ATCC 13869, and an amino acid sequence of CarB may be ANU33812.1 derived from Corynebacterium glutamicum ATCC 13869, but it is obvious that proteins with CarA or CarB activity, derived from various origins, are comprised.

In the present disclosure, CarA may have, comprise, or consist of an amino acid sequence represented by SEQ ID NO: 1, or may essentially consist of the amino acid sequence, and CarB may have, comprise, or consist of an amino acid sequence represented by SEQ ID NO: 2, or may essentially consist of the amino acid sequence.

In the present disclosure, the amino acid sequence of SEQ ID NO: 1 and/or SEQ ID NO: 2 may comprise an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1 and/or SEQ ID NO: 2. It is also apparent that any protein having an amino acid sequence with deletion, modification, substitution, conservative substitution or addition in part of the sequence may also fall within the scope of the present disclosure, as long as the amino acid sequence has such a homology or identity and exhibits efficacy corresponding to that of the protein comprising the amino acid sequence of SEQ ID NO: 1 and/or SEQ ID NO: 2.

Examples thereof comprise those having sequence addition or deletion that do not alter the function of the protein of the present disclosure at the N-terminus, C-terminus of the amino acid sequence, and/or inside the amino acid sequence, a naturally occurring mutation, a silent mutation, or a conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of residues. Usually, conservative substitution may hardly affect or not affect the activity of the proteins or polypeptides.

As used herein, the term 'homology' or 'identity' means the degree of similarity between two given amino acid sequences or nucleotide sequences and may be expressed as a percentage. The terms 'homology' and 'identity' may often be used interchangeably.

The sequence homology or identity of a conserved polynucleotide or polypeptide is determined by standard alignment algorithms, and the default gap penalty established by the used program may be used together. Substantially, homologous or identical sequences are generally capable of being hybridized with the entirety or a part of the sequence under moderately or highly stringent conditions. It is apparent that hybridization also comprises hybridization with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al (1988)[Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL/.](1988) SIAM J Applied Math 48: 1073). For example, BLAST of the National Center for Biotechnology Information, or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may comprise (1) a binary comparison matrix (comprising values of 1 for identity and 0 for non-identity) and a weighted comparison matrix of Gribskov et al(1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

The CarA and CarB of the present disclosure may be encoded by a carAB operon gene.

For example, the carA gene may be a polynucleotide encoding ANU33813.1 derived from *Corynebacterium glutamicum* ATCC 13869, and the carB gene may be a polynucleotide encoding ANU33812.1 derived from *Corynebacterium glutamicum ATCC* 13869, but are not limited thereto. It is obvious that the present disclosure comprises carA and/or carB genes derived from various origins, which encode proteins having CarA and/or CarB activities.

As used herein, the term "polynucleotide", which is a polymer of nucleotides, in which nucleotide monomers are linked in a long chain shape by covalent bonds, refers to a DNA or RNA strand having a predetermined or longer length, more specifically, a polynucleotide fragment encoding the protein.

The polynucleotide encoding the carbamoyl phosphate synthetase of the present disclosure may comprise a nucleotide sequence encoding the amino acid sequence represented by SEQ ID NO: 1 and/or SEQ ID NO: 2. In one embodiment of the present disclosure, the polynucleotide of the present disclosure may have or comprise a sequence of SEQ ID NO: 3. Further, the polynucleotide of the present disclosure may consist of or essentially consist of the sequence of SEQ ID NO: 3. Specifically, the carbamoyl phosphate synthetase may be encoded by the polynucleotide represented by the nucleotide sequence of SEQ ID NO: 3.

In the polynucleotide of the present disclosure, various modifications may be made in the coding region as long as the amino acid sequence of carbamoyl phosphate synthetase is not changed, in consideration of codon degeneracy or codons preferred in organisms that are intended to express the carbamoyl phosphate synthetase of the present disclosure. Specifically, the polynucleotide of the present disclosure may have or comprise a nucleotide sequence having 70% or more, 75% or more, 76% or more, 85% or more, 90% or more, 95% or more, 96% or more, 97% or more, 98% or more homology or identity to the sequence of SEQ ID NO: 3, or may consist of or essentially consist of a nucleotide sequence having 70% or more, 75% or more, 76% or more, 85% or more, 90% or more, 95% or more, 96% or more, 97% or more, 98% or more homology or identity to the sequence of SEQ ID NO: 3, but is not limited thereto.

Further, the polynucleotide of the present disclosure may comprise a probe that may be prepared from a known gene sequence, for example, any sequence without limitation as long as it is a sequence that is able to hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York, 9.50-9.51, 11.7-11.8). Examples thereof comprise conditions under which polynucleotides having higher homology or identity, namely polynucleotides having 70% or more, 75% or more, 76% or more, 85% or more, 90% or more, 95% or more, 96% or more, 97% or more, 98% or more, or 99% or more homology or identity are hybridized with each other while polynucleotides having lower homology or identity are not hybridized with each other, or conditions under which washing is performed once, specifically twice to three times at a salt concentration and temperature equivalent to 60°C, 1×SSC, 0.1% SDS, specifically 60°C, 0.1×SSC, 0.1% SDS, more specifically 68°C, 0.1×SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relationship between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

As used herein, the term "microorganism (or strain)" comprises all wild-type microorganisms or naturally or artificially genetically modified microorganisms, and it may be a microorganism in which a specific mechanism is weakened or strengthened due to an insertion of a foreign gene or an activity enhancement or inactivation of an endogenous gene, and it may be a microorganism comprising a genetic modification for the production of a target polypeptide, protein, or product.

In one embodiment of the present disclosure, the microorganism of the present disclosure may be a microorganism, in which the activity of carbamoyl phosphate synthetase is weakened. The microorganism may be a microorganism, in which the activity of any one or more selected from the group consisting of CarA and CarB is weakened. For example, the microorganism of the present disclosure may be a microorganism, in which CarA and CarB or the carAB operon gene encoding the same are weakened, as compared to endogenous activity, but is not limited thereto.

As used herein, the term "weakening" of the activity of the polypeptide (e.g., protein specified by the name of each enzyme) has a concept encompassing all of the decrease of activity or the absence of activity, as compared to the endogenous activity. The weakening may be used interchangeably with terms such as inactivation, deficiency, down-regulation, decrease, reduction, attenuation, etc.

The weakening may also comprise a case where the activity of the polypeptide itself is decreased or eliminated due to a variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or due to inhibition of translation into the polypeptide, as compared to that of the natural strain, a case where the polynucleotide is not expressed at all, and/or a case where the polypeptide activity is absent even when the polynucleotide is expressed. The "endogenous activity" means the activity of a specific polypeptide originally possessed by a parent strain before the trait is changed or a wild-type or unmodified microorganism, when the trait is changed by genetic variation due to natural or artificial factors. This may be used interchangeably with "activity before modification". The "weakening, inactivation, deficiency, decrease, down-regulation, reduction, or attenuation" of the activity of a polypeptide compared to the endogenous activity thereof means that the activity of the polypeptide is lowered, compared to the activity of a specific polypeptide originally possessed by a parent strain before the trait is changed or an unmodified microorganism.

With respect to the objects of the present disclosure, the microorganism of the present disclosure is a microorganism having improved L-amino acid-producing ability by having weakened carbamoyl phosphate synthetase activity, and an unmodified microorganism with unweakened carbamoyl phosphate synthetase, which is a target strain for comparing whether or not the L-amino acid-producing ability increases, may be a wild-type *Corynebacterium glutamicum* ATCC 13869 strain, a *Corynebacterium glutamicum* ATCC 13869 strain with weakened ArgF protein activity, which is known as an L-ornithine producing strain, or a *Corynebacterium glutamicum* ATCC 13869 strain with weakened ArgR protein activity, but is not limited thereto.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be achieved by:
1) deletion of the entirety or a part of the gene encoding the polypeptide;
2) modification of an expression control region (or expression control sequence) to reduce the expression of a gene encoding the polypeptide;
3) modification of the amino acid sequence constituting the polypeptide to eliminate or weaken the activity of the polypeptide (e.g., deletion/substitution/addition of one or more amino acids in the amino acid sequence);
4) modification of the gene sequence encoding the polypeptide to eliminate or weaken the activity of the polypeptide (e.g., deletion/substitution/addition of one or more nucleotides in the nucleotide sequence of the polypeptide gene to encode the polypeptide which is modified to eliminate or weaken the activity of the polypeptide);
5) modification of a nucleotide sequence encoding an initiation codon, Shine-Dalgarno sequence, or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) combination of two or more selected from 1) to 8), but is not particularly limited thereto.

For example,
1) The deletion of a part or the entirety of the gene encoding the polypeptide may be elimination of the entirety of the polynucleotide encoding an endogenous target polypeptide in the chromosome, replacement with a polynucleotide having deletion of some nucleotides, or replacement with a marker gene.
2) The modification of an expression control region (or expression control sequence) may be the mutation on the expression control region (or expression control sequence) through deletion, insertion, non-conservative or conservative substitution, or a combination thereof, or the replacement with a sequence having weaker activity. The expression control region comprises a promoter, an operator sequence, a sequence for encoding a ribosomal binding site, and a sequence for controlling the termination of transcription and translation, but is not limited thereto.
3) and 4) The modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence improved to have weaker activity, or an amino acid sequence or polynucleotide sequence improved to have no activity, so as to weaken activity of the polypeptide, but is not limited thereto. For example, the expression of the gene may be inhibited or attenuated by introducing mutation into the polynucleotide sequence to form a stop codon, but is not limited thereto.
5) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.
6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.

Further, 8) the addition of a promoter, which is to be reverse-transcribed, to the 3' end of the open reading frame (ORF) of the gene sequence encoding the polypeptide (reverse transcription engineering, RTE) may be making an antisense nucleotide complementary to the gene transcript encoding the polypeptide to weaken the activity.

For example, the activity of carbamoyl phosphate synthetase of the present disclosure may be weakened by the modification of the initiation codon of the gene transcript encoding carbamoyl phosphate synthetase, and specifically, may be weakened by the substitution of the initiation codon of the carAB operon gene transcript encoding carbamoyl phosphate synthetase with another initiation codon having a lower expression rate of the polypeptide. For another example, the activity of carbamoyl phosphate synthetase of the present disclosure may be weakened by the modification of a promoter of the gene encoding carbamoyl phosphate synthetase, or by replacement with a weakened promoter, but is not limited thereto.

The modification of a part or the entirety of the polynucleotide in the microorganism of the present disclosure may be induced by: (a) homologous recombination using a vector for chromosomal insertion in a microorganism or genome editing using engineered nuclease (e.g., CRISPR-Cas9), and/or (b) treatment with light, such as ultraviolet light and radiation, and/or chemicals, but is not limited thereto. The method of modifying a part or the entirety of the gene may comprise a method by DNA recombinant technology. For example, a nucleotide sequence or vector comprising a nucleotide sequence homologous to a target gene may be introduced into the microorganism to bring about homologous recombination, resulting in the deletion of a part or the entirety of the gene. The nucleotide sequence or vector to be introduced may comprise a dominant selection marker, but is not limited thereto.

The vector of the present disclosure may comprise a DNA construct comprising a nucleotide sequence of a polynucleotide encoding a desired polypeptide that is operably linked to a suitable expression regulatory region (or expression control sequence) so that the desired polypeptide may be expressed in a suitable host. The expression regulatory region may comprise a promoter capable of initiating transcription, any operator sequence for controlling transcription, a sequence encoding a suitable mRNA ribosome binding site, and a sequence controlling the termination of transcription and translation. The vector may be transformed into a suitable host cell, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2, pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a desired polypeptide. The polynucleotide may be introduced in any form as long as it may be introduced into a host cell and expressed. For example, the polynucleotide may be introduced into a host cell in the form of an expression cassette, which is a gene construct comprising all elements required for autonomous expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the polynucleotide sequence is functionally linked to a promoter sequence that initiates and mediates transcription of the polynucleotide encoding the desired polypeptide of the present disclosure.

In another embodiment of the present disclosure, the microorganism of the present disclosure may be a microorganism having L-amino acid-producing ability.

The microorganism of the present disclosure may be a microorganism having improved L-amino acid-producing ability.

The microorganism of the present disclosure may be a microorganism in which the carbamoyl phosphate synthetase activity is weakened. In the microorganism, the activity of any one or more selected from the group consisting of CarA and CarB may be weakened. Specifically, the microorganism of the present disclosure may be a microorganism in which CarA and CarB or the carAB operon gene encoding the same are weakened; or a microorganism (e.g., recombinant microorganism) that is genetically modified to weaken CarA and CarB or the carAB operon gene encoding the same, but is not limited thereto.

The microorganism of the present disclosure may be a microorganism naturally having L-amino acid-producing ability or a microorganism provided with L-amino acid-producing ability by weakening the carbamoyl phosphate synthetase or the polynucleotide encoding the same in a parent strain having no L-amino acid-producing ability, but is not limited thereto.

With respect to the objects of the present disclosure, the recombinant microorganism of the present disclosure may be a microorganism in which the L-amino acid-producing ability is increased by weakening the carbamoyl phosphate synthetase or the polynucleotide encoding the same in the natural wild-type microorganism or in the microorganism producing L-amino acids by comprising the carbamoyl phosphate synthetase or the polynucleotide encoding the same, as compared to a natural wild-type microorganism or a microorganism producing L-amino acids by comprising carbamoyl phosphate synthetase or the polynucleotide encoding the same, but is not limited thereto. For example, the unmodified microorganism with unweakened carbamoyl phosphate synthetase, which is a target strain for comparing whether or not the L-amino acid-producing ability increases, may be a wild-type *Corynebacterium glutamicum* ATCC 13869 strain, a *Corynebacterium glutamicum* ATCC 13869 strain with weakened ArgF protein activity, which is known as an L-ornithine producing strain, or a *Corynebacterium glutamicum* ATCC 13869 strain with weakened ArgR protein activity, but is not limited thereto.

For example, the recombinant strain having the increased production ability may have an increased L-amino acid-producing ability of about 1% or more, specifically, about 2% or more, about 5% or more, about 6% or more, about 7% or more, about 8% or more, about 9% or more, about 10% or more, about 13% or more, about 15% or more, about 16% or more, about 17% or more, about 18% or more, about 19% or more, about 20% or more, about 21% or more, about 24% or more, about 25% or more, about 27% or more, about 30% or more, about 31% or more, about 35% or more, about 40% or more, about 45% or more, about 49% or more, about 50% or more, about 55% or more, about 60% or more, about 65% or more, about 70% or more, about 75% or more, about 80% or more, about 85% or more, about 90% or more, about 95% or more, or about 97% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or the unmodified microorganism, but the increased amount is not limited thereto as long as the production ability has an increased amount of a + value, as compared to the production ability of the parent strain before modification or the unmodified microorganism. In another example, the microorganism having the increased production ability may have an increased L-amino acid-producing ability of about 1.01 times or more, about 1.02 times or more, about 1.05 times or more, about 1.06 times or more, about 1.07 times or more, about 1.08 times or more, about 1.09 times or more, about 1.1 times or more, about 1.13 times or more, about 1.15 times or more, about 1.16 times or more, about 1.17 times or more, about 1.18 times or more, about 1.19 times or more, about 1.20 times or more, about 1.21 times or more, about 1.24 times or more, about 1.25 times or more, about 1.27 times or more, about 1.30 times or more, about 1.31 times or more, about 1.35 times or more, about 1.40 times or more, about 1.45 times or more, about 1.49 times or more, about 1.50 times or more, about 1.55 times or more, about 1.60 times or more, about 1.65 times or more, about 1.70 times or more, about 1.75 times or more, about 1.80 times or more, about 1.85 times or more, about 1.90 times or more, about 1.95 times or more, or about 1.97 times or more (the upper limit is not particularly limited, but may be, for example, about 10 times or less, about 5 times or less, about 3 times or less, or about 2 times or less), as compared to that of the parent strain before modification or the unmodified microorganism, but is not limited thereto.

As used herein, the term "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may refer to a strain in which the carbamoyl phosphate synthetase described herein or the polynucleotide encoding the same is not weakened or has not yet been weakened. The "unmodified microorganism" may be used interchangeably with "strain before being modified", "microorganism before being modified", "unvaried strain", "unmodified strain", "unvaried microorganism", or "reference microorganism".

In the present disclosure, the L-amino acid may be any one or more selected from the group consisting of L-ornithine, L-glutamate, L-aspartate, and L-phenylalanine.

In another embodiment of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium glutamicum,* but is not limited thereto.

In still another embodiment of the present disclosure, the recombinant microorganism of the present disclosure may be a microorganism in which the L-amino acid-producing ability is enhanced by additionally enhancing the activity of a part of proteins in the L-amino acid biosynthetic pathway or by additionally weakening the activity of a part of proteins in the L-amino acid degradation pathway.

Specifically, the microorganism of the present disclosure may be a microorganism in which the activity of ornithine carbamoyltransferase subunit F (ArgF) is additionally weakened, or the argF gene encoding the same is additionally deleted, and/or the activity of arginine repressor (ArgR) is additionally weakened, or the argR gene encoding the same is additionally deleted.

An amino acid sequence of the ArgF or ArgR may be obtained from NCBI's Genbank which is a known database, etc. For example, the amino acid sequence of ArgF of the present disclosure may comprise ANU33618.1 (SEQ ID NO: 19) derived from *Corynebacterium glutamicum* ATCC 13869 or an amino acid sequence having 90% or more homology thereto, and the amino acid sequence of ArgR may comprise ANU33619.1 (SEQ ID NO: 24) derived from *Corynebacterium glutamicum* ATCC 13869 or an amino acid sequence having 90% or more homology thereto, but is not limited thereto, and it is obvious that proteins with ArgF or ArgR activity, derived from various origins, are comprised.

However, the weakening of the ArgF and/or ArgR protein activity or the deletion of the argF and/or argR gene is only one example and is not limited thereto, and the microorganism of the present disclosure may be a microorganism in which the activities of the proteins in various known L-amino acid biosynthetic pathways are enhanced or the activities of the proteins in the degradation pathway are weakened.

As used herein, the term "enhancement" of the polypeptide activity means that the activity of the polypeptide is increased, as compared to the endogenous activity thereof. The enhancement may be used interchangeably with terms such as activation, up-regulation, overexpression, increase, etc. Here, the activation, enhancement, up-regulation, overexpression, and increase may comprise both cases in which an activity not originally possessed is exhibited, or the activity is enhanced, as compared to the endogenous activity or the activity before modification. The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification". The fact that the activity of a polypeptide is "enhanced", "up-regulated", "overexpressed", or "increased, as compared to the endogenous activity" means that the activity of the polypeptide is improved as compared to the activity and/or concentration (expression level) of the specific polypeptide originally possessed by a parent strain before the trait is changed or an unmodified microorganism.

The enhancement may be achieved through the introduction of a foreign polypeptide or the enhancement of the activity of the endogenous polypeptide, and/or concentration (expression level). The enhancement of the activity of the polypeptide may be confirmed by an increase in the degree of activity and the expression level of the corresponding polypeptide or an increase in the amount of the product released from the corresponding polypeptide.

For the enhancement of the activity of the polypeptide, various methods well known in the art may be applied, and the method is not limited as long as the activity of the desired polypeptide may be enhanced as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence having stronger activity;
3) modification of a nucleotide sequence encoding an initiation codon or 5'-UTR region of the gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

More specifically, 1) the increase in the intracellular copy number of the polynucleotide encoding the polypeptide may be achieved by introducing, into a host cell, a vector which may replicate and function independently of the host and to which the polynucleotide encoding the corresponding polypeptide is operably linked. Alternatively, the increase may be achieved by introducing one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.

2) The replacement of a gene expression regulatory region (or expression control sequence) on a chromosome encoding the polypeptide with a sequence exhibiting strong activity may be, for example, occurrence of variation in a sequence due to deletion, insertion, nonconservative or conservative substitution, or a combination thereof, or replacement with a sequence exhibiting stronger activity so that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.

Examples of known strong promoters comprise CJ1 to CJ7 promoters (US 7662943 B2), a lac promoter, a trp promoter, a trc promoter, a tac promoter, a lambda phage PR promoter, a PL promoter, a tet promoter, a gapA promoter, a SPL7 promoter, a SPL13(sm3) promoter (US 10584338 B2), an O2 promoter (US 10273491 B2), a tkt promoter, an yccA promoter, etc., but is not limited thereto.

3) The modification of a nucleotide sequence encoding an initiation codon or 5'-UTR region of the gene transcript encoding the polypeptide may be, for example, substitution with a nucleotide sequence encoding another initiation codon having a higher polypeptide expression rate as compared to an endogenous initiation codon, but is not limited thereto.

4) and 5) The modification of the amino acid sequence or the polynucleotide sequence may be occurrence of variation in the sequence due to deletion, insertion, nonconservative or conservative substitution of the amino acid sequence of the polypeptide or the polynucleotide sequence encoding the polypeptide, or a combination thereof, or replacement with an amino acid sequence or polynucleotide sequence improved to exhibit stronger activity or an amino acid sequence or polynucleotide sequence improved to be more active so that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion.

6) The introduction of a foreign polynucleotide exhibiting the activity of the polypeptide may be introduction of a foreign polynucleotide into a host cell, the foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in the host cell, the polypeptide may be produced and the activity thereof may be increased.

7) The codon optimization of the polynucleotide encoding the polypeptide may be codon optimization of an endogenous polynucleotide so as to enhance transcription or translation in a host cell, or codon optimization of a foreign polynucleotide so as to perform optimized transcription and translation in a host cell.

8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration/expression level of the corresponding polypeptide, based on the activity or concentration of the polypeptide expressed in a wild-type or a microbial strain before being modified, or an increase in the amount of a product produced from the corresponding polypeptide, but is not limited thereto.

Another aspect of the present disclosure provides a method of producing L-amino acids, the method comprising the step of culturing, in a medium, the microorganism of the genus *Corynebacterium* having L-amino acid-producing ability, in which the activity of carbamoyl phosphate synthetase is weakened.

The method of producing L-amino acids of the present disclosure may comprise the step of culturing, in a medium, a microorganism in which CarA and CarB or the carAB operon gene encoding the same are weakened; or a microorganism that is genetically modified to weaken CarA and CarB or the carAB operon gene encoding the same, and the microorganism is as described above.

As used herein, the term "culturing" refers to growing the microorganism of the present disclosure in appropriately adjusted environmental conditions. The culture procedure of the present disclosure may be performed according to appropriate media or culture conditions known in the art. Such a culture procedure may be easily adjusted according to the selected strain by a person skilled in the art. Specifically, the culturing may be in a batch type, a continuous type, and/or a fed-batch type, but is not limited thereto.

As used herein, the "medium" refers to a mixture comprising, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the microorganism of the present disclosure, any medium that is used for the usual culture of microorganisms may be used without particular limitation. However, the microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

Specifically, the culture medium for the microorganism of the genus *Corynebacterium* may be found in the literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fish or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected, or nitrogen, hydrogen, or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to about 160 hours, but is not limited thereto.

The L-amino acid produced by the culturing of the present disclosure may be released into the medium or may remain in the cells.

The L-amino acid may be any one or more selected from the group consisting of L-ornithine, L-glutamate, L-aspartate, and L-phenylalanine.

The method of producing the L-amino acid of the present disclosure may further comprise the steps of preparing the microorganism of the present disclosure, preparing a medium for culturing the microorganism, or a combination thereof (regardless of the order, in any order), for example, before the culturing step.

The method of producing the L-amino acid of the present disclosure may further comprise the step of recovering the L-amino acid from a medium resulting from the culturing (a medium in which culturing has been performed) or the cultured microorganism. The recovering step may be further comprised after the culturing step.

The recovering may involve collecting the desired L-amino acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, etc., HPLC, and a combination of these methods may be used, and the desired L-amino acid may be recovered from the medium or microorganism by using an appropriate method known in the art.

In addition, the method of producing the L-amino acid of the present disclosure may further comprise a purification step. The purification may be performed by using an appropriate method known in the art. In an exemplary embodiment, when the method of producing the L-amino acid of the present disclosure comprises both the recovering step and the purification step, the recovering step and the purification step may be performed continuously or discontinuously regardless of the order, or may be performed simultaneously or integrated into one step, but is not limited thereto.

With regard to the method of the present disclosure, the carbamoyl phosphate synthetase, the polynucleotide, the vector, and the microorganism, etc. are as described in other aspects.

Still another aspect of the present disclosure provides a composition for producing L-amino acids, the composition comprising the microorganism of the genus *Corynebacterium,* in which the carbamoyl phosphate synthetase is weakened; a medium in which the microorganism is cultured; or a combination thereof.

The composition of the present disclosure may further comprise any appropriate excipient that is usually used in the composition for producing L-amino acids, and such excipients may comprise, for example, a preserving agent, a wetting agent, a dispersing agent, a suspending agent, a buffering agent, a stabilizing agent, an isotonic agent, etc., but are not limited thereto.

Still another aspect of the present disclosure provides a method of preparing the microorganism of the genus *Corynebacterium* having L-amino acid-producing ability, the method comprising the step of weakening carbamoyl phosphate synthetase.

Still another aspect of the present disclosure provides use of the microorganism of the genus *Corynebacterium,* in which activity of carbamoyl phosphate synthetase is weakened, in producing L-amino acids.

The carbamoyl phosphate synthetase, the weakening, the microorganism of the genus *Corynebacterium,* etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1: Construction of vector

### Example 1-1: Construction of vector for replacement of carAB promoter

A vector for replacing a promoter of carA gene encoding carbamoyl phosphate synthase small subunit (CarA) was constructed. Information (NZ_CP016335.1) about carAB gene (SEQ ID NO: 3) encoding CarA (ANU33813.1, SEQ ID NO: 1) and its surrounding nucleotide sequence was obtained from NIH GenBank, and was used to construct the vector for replacing the promoter region of carA with a promoter (SEQ ID NO: 4) of betP(ANU33153.1). The genome of wild-type *C. glutamicum* ATCC 13869 was used as a template, and betP promoter, homologous recombinant A arm, and homologous recombinant B arm were amplified by PCR (Sol^{™} Pfu-X DNA polymerase) using a primer pair of SEQ ID NOS: 5 and 6, a primer pair of SEQ ID NOS: 7 and 8, a primer pair of SEQ ID NOS: 9 and 10, respectively, thereby obtaining respective DNA fragments.

The obtained promoter and recombinant arm gene fragments were cloned into a vector pDCM2 (Korean Patent Publication No. 10-2020-0136813) digested with Sall and BamHI restriction enzymes using Gibson assembly (DG Gibson et al., NATURE METHODS, VOL.6 NO.5, MAY 2009, NEBuilder HiFi DNA Assembly Master Mix) to construct the vector. The constructed vector was transformed into *E. coli* DH5α, and plated on an LB solid medium comprising kanamycin (25 mg/l). To select colonies transformed with the vector, PCR was performed using a primer pair of SEQ ID NOS: 11 and 12, and a plasmid was obtained from the selected colonies using a plasmid extraction method commonly known. The obtained plasmid was named pDCM2-PbetP-carA.

### Example 1-2: Construction of vector for mutation of carA initiation codon

A vector for replacing the initiation codon gtg of gene carA with ttg was constructed. The genome of wild-type *C. glutamicum* ATCC 13869 was used as a template, and homologous recombinant A arm was amplified using a primer pair of SEQ ID NOS: 7 and 13, and homologous recombinant B arm was amplified using a primer pair of SEQ ID NOS: 10 and 14. Thereafter, a plasmid was obtained in the same manner as above, and named pDCM2-carA(g1t).

### Example 1-3: Construction of vector for mutation of carB initiation codon

A vector for replacing the initiation codon atg of gene carB with ttg was constructed. Information (NZ_CP016335.1) about carAB gene (SEQ ID NO: 3) encoding carbamoyl phosphate synthetase large subunit (SEQ ID NO: 2) and its surrounding nucleotide sequence was obtained from NIH GenBank. The genome of wild-type *C. glutamicum* ATCC 13869 was used as a template, and homologous recombinant A arm and homologous recombinant B arm were amplified using a primer pair of SEQ ID NOS: 15 and 16 and a primer pair of SEQ ID NOS: 17 and 18, respectively. Thereafter, a plasmid was obtained in the same manner as above, and named pDCM2-carB(a1t).

The sequences of the primers used in Examples 1-1 to 1-3 are as in Table 1 below.

**[Table 1]**

| SEQ ID NO. | Sequence name | Sequence (5'-> 3') |
|---|---|---|
| 5 | primer 2 | GGCACAAGATGGGGTGCGTGGCGAGATCACC GTT |
| 6 | primer 3 | TATCGGTTTCGAATTTGGGTCAGATGTAGTCAT A |
| 7 | primer 4 | CGGTACCCGGGGATCCCAAGAAGGCGCTTCTC G |
| 8 | primer 5 | GCACCCCATCTTGTGCCGGTGACTGTTGTCGC CT |
| 9 | primer 6 | CAAATTCGAAACCGATAGTGAGTAAAGACACCA C |
| 10 | primer 7 | ATGCCTGCAGGTCGACGAGACCTCAACGGAGA G |
| 11 | primer 8 | TATTACGCCAGCTGGCGAAA |
| 12 | primer 9 | GCTTTACACTTTATGCTTCC |
| 13 | primer 10 | GGTGACTGTTGTCGCCTTTCGTGTGTCTGTC |
| 14 | primer 11 | GGCGACAACAGTCACCTTGAGTAAAGACACCAC |
| 15 | primer 12 | CGGTACCCGGGGATCCCCCACGTCGTTTCTCT GCAC |
| 16 | primer 13 | GTTATTTATGC GC CTTTCTTC |
| 17 | primer 14 | GAAAGGCGCATAAATAACTTGCCAAAGCGTTCA GATAT |
| 18 | primer 15 | ATGCCTGCAGGTCGACGCCACCCATAGTGAAG GATG |

### Example 2: Preparation of strain producing L-ornithine

### Example 2-1: Construction of vector for ArgF inactivation

To prepare a strain producing L-ornithine, a vector was constructed, in which the vector replaces serine at position 55 of a protein sequence of ArgF (ANU33618.1, SEQ ID NO: 19) with a stop codon. The genome of wild-type C. *glutamicum* ATCC1 13869 was used as a template, and homologous recombinant A arm and homologous recombinant B arm were amplified using a primer pair of SEQ ID NOS: 20 and 21 and a primer pair of SEQ ID NOS: 22 and 23, respectively. Thereafter, a plasmid was obtained in the same manner as above, and named pDCM2-argF(S55*).

### Example 2-2: Construction of vector for ArgR inactivation

A vector was constructed, in which the vector replaces glutamate at position 47 of a protein sequence of ArgR (ANU33619.1, SEQ ID NO: 24) with a stop codon. The genome of wild-type *C. glutamicum ATCC* 13869 was used as a template, and homologous recombinant A arm and homologous recombinant B arm were amplified using a primer pair of SEQ ID NOS: 25 and 26 and a primer pair of SEQ ID NOS: 27 and 28, respectively. Thereafter, a plasmid was obtained in the same manner as above, and named pDCM2-argR(E47*).

The sequences of the primers used in Examples 2-1 to 2-2 are as in Table 2 below.

**[Table 2]**

| SEQ ID NO. | Sequence name | Sequence (5'-> 3') |
|---|---|---|
| 20 | primer 16 | CGGTACCCGGGGATCCTGACCCCAGGCAAGC ACGG |
| 21 | primer 17 | GAAGCGAGTACGAGTTTAAGTCTTATC |
| 22 | primer 18 | AAACTCGTACTCGCTTCTCC |
| 23 | primer 19 | ATGCCTGCAGGTCGACCGGCGCCGGCAACCT CGTC |
| 25 | primer 20 | CGGTACCCGGGGATCCCTCGTGCGGAATTCG TGGAG |
| 26 | primer 21 | ATCCAGCAGCAATTCAGACA |
| 27 | primer 22 | CTGAATTGCTGCTGGATTAAGGCATCGATATCA CCCA |
| 28 | primer 23 | ATGCCTGCAGGTCGACCCTTCATTTTAAGTTCC TTG |

### Example 2-3: Preparation of strain producing L-ornithine

To prepare a strain producing L-ornithine, wild-type *C*. *glutamicum* ATCC 13869 was transformed with the pDCM2-argF(S55*) vector constructed in Example 2-1 by electroporation (Appl. Microbiol.Biotechnol. (1999) 52:541-545), and then a second crossover process was performed to obtain a strain, in which a nucleotide sequence at position 164 of argF was substituted from cytosine (c) to adenine (a) and thus a protein sequence at position 55 was substituted with a stop codon. PCR and sequencing analysis were performed using a primer pair of SEQ ID NOS: 20 and 23 which are capable of amplifying the adjacent region comprising the position where the corresponding gene was inserted, and the corresponding genetic manipulation was confirmed. The strain thus obtained was named C. g I: : arg F*.

To prepare a strain in which ornithine production was further improved in C. gl::argF*, the pDCM2-argR(E47*) vector prepared in Example 2-2 was used to obtain a strain in the same manner as above. PCR and sequencing analysis were performed using a primer pair of SEQ ID NOS: 25 and 28 which are capable of amplifying the adjacent region comprising the position where the corresponding gene was inserted, and the corresponding genetic manipulation was confirmed. The strain thus obtained was named C. gl::argF*_argR*.

### Example 3: Preparation of CarAB-weakened strain

The pDCM2-PbetP-carA and pDCM2-carA(g1t) vectors constructed in Examples 1-1 to 1-2 were transformed into wild-type *C. glutamicum* ATCC 13869 and C. gl::argF*and C. gl::argF*_argR* prepared in Example 2-3 by electroporation, respectively, and strains were obtained through a second crossover process. PCR and sequencing analysis were performed using a primer pair of SEQ ID NOS: 6 and 9, and the corresponding genetic manipulation was confirmed. The strains thus obtained were named in this order: C. gl::PbetP-carA, C. gl::carA(g1t), C. gl::argF*_PbetP-carA, C. gl::argF*_carA(g1t), C. gl::argF*_argR*_PbetP-carA, C. gl::argF*_argR*_carA(g1t).

To prepare strains, in which carB promoter and initiation codon were replaced, the pDCM2-carB(a1t) vector which was the plasmid obtained in Example 1-3 was transformed into wild-type *C. glutamicum* ATCC 13869, C. gl::argF*, C. gl::argF*_argR* by electroporation, respectively, and strains were obtained through a second crossover process. PCR and sequencing analysis were performed using a primer pair of SEQ ID NOS: 16 and 19 which are capable of amplifying the adjacent region comprising the position where the corresponding gene was inserted, and the corresponding genetic manipulation was confirmed. The strains thus obtained were named in this order: C. gl::carB(a1t), C. gl::argF*_carB(a1t), C. gl::argF*_argR*_carB(a1t).

### Example 4: Evaluation of L-amino acid-producing ability of recombinant strains

The C. gl::PbetP-carA, C. gl::carA(g1t), C. gl::carB(a1t) strains prepared in Example 3 and a control wild-type *C. glutamicum* strain were cultured to compare the cell mass and L-amino acid-producing ability.

First, each strain was inoculated into a 250 ml corner-baffled flask comprising 25 ml of a seed medium, and cultured at 30°C for 16 hours with shaking at 200 rpm. 1 ml of the seed culture was inoculated into a 250 ml corner-baffled flask comprising 24 ml of a production medium, and cultured at 33°C for 40 hours with shaking at 200 rpm.

After the culture was completed, the production of 20 types of L-amino acids was measured using HPLC.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, 2 mg of nicotinamide (based on 1 liter of distilled water)

### <Production medium (pH 7.0)>

50 g of raw sugar, 25 g of (NH₄)₂SO₄, 1 g of yeast extract, 0.55 g of KH₂PO₄, 0.6 g of MgSO₄·7H₂O, 0.9 mg of biotin, 4.5 mg of thiamine HCl, 4.5 mg of calcium pantothenate, 30 mg of nicotinamide, 9 mg of MnSO₄, 9 mg of FeSO₄, 0.45 mg of ZnSO₄, 0.45 mg of CuSO₄, 30 g of CaCOs (based on 1 liter of distilled water)

The above experiment was repeated three times, and the culture results (average value) are shown in Table 3 below. The increase rate of the concentration of each L-amino acid was calculated by (experimental group-control group)/control group.

**[Table 3]**

| Strain | OD₅₆₂ | Sugar consu mption (g/L) | L-glutam ate concen tration (mg/L) | Increase rate of L-glutamat e concentr ation (%) | L-asp arta te con cent rati on (mg /L) | Increase rate of L-aspartate concentra tion (%) | L-phenyl alanin e conce ntratio n (mg/L) | Increase rate of L-phenylal anine concentr ation (%) |
|---|---|---|---|---|---|---|---|---|
| Wild-type C. *glutamicum* ATCC 13869 | 95.6 | 50.0 | 680.3 | - | 33. 7 | - | 0.00 | - |
| C. gl::PbetP-carA | 94.4 | 50.0 | 888.2 | 31 | 50. 1 | 49 | 20.7 | 21 |
| C. gl::carA(g1t) | 94.7 | 50.0 | 841.0 | 24 | 66. 5 | 97 | 18.3 | 18 |
| C. gl::carB(a1t) | 94.9 | 50.0 | 867.3 | 27 | 64. 1 | 90 | 20.2 | 20 |

As shown in Table 3, C. gl::PbetP-carA, C. gl::carA(g1t), and C. gl::carB(a1t) strains showed increased L-glutamate, L-aspartate, and L-phenylalanine production and yields, as compared to the control wild-type *C. glutamicum* ATCC 13869 strain.

### Example 5: Evaluation of L-ornithine-producing ability of recombinant strains

C. gl::argF*_PbetP-carA, C. gl::argF*_carA(g1t), C. gl::argF*_carB(a1t), C. gl::argF*_argR*_PbetP-carA, C. gl::argF*_argR*_carA(g1t), C. gl::argF*_argR*_carB(a1t) strains prepared in Example 3, and control strains C. gl::argF*, C. gl::argF*_argR* were cultured using the media below in the same manner as in Example 4 to compare the cell mass and L-ornithine-producing ability.

### <Seed medium (pH 7.0)>

20 g of glucose, 10 g of peptone, 5 g of yeast extract, 1.5 g of urea, 4 g of KH₂PO₄, 8 g of K₂HPO₄, 0.5 g of MgSO₄·7H₂O, 0.1 mg of biotin, 1 mg of thiamine HCl, 22 mg of calcium pantothenate, 2 mg of nicotinamide (based on 1 liter of distilled water)

### <Production medium (pH 7.0)>

50 g of raw sugar, 25 g of (NH₄)₂SO₄, 1 g of yeast extract, 0.55 g of KH₂PO₄, 0.6 g of MgSO₄·7H₂O, 0.2 g of L-arginine, 0.9 mg of biotin, 4.5 mg of thiamine HCl, 4.5 mg of calcium pantothenate, 30 mg of nicotinamide, 9 mg of MnSO₄, 9 mg of FeSO₄, 0.45 mg of ZnSO₄, 0.45 mg of CuSO₄, 30 g of CaCOs (based on 1 liter of distilled water)

The above experiment was repeated three times, and the culture results (average value) are shown in Table 4 below. The L-ornithine yield was calculated by ornithine concentration/sugar consumption, and the concentration increase rate was calculated by (concentration of L-ornithine produced by the experimental group-concentration of L-ornithine produced by the control group (wild-type C. *glutamicum* ATCC 13869 strain))/concentration of L-ornithine produced by the control group (wild-type *C. glutamicum* ATCC 13869 strain).

**[Table 4]**

| Strain | OD₅₆₂ | Sugar consum ption (g/L) | L-ornithine concentratio n (g/L) | L-ornithine yield (%) | Increase rate of L-ornithine concentration (%) |
|---|---|---|---|---|---|
| Wild-type *C. glutamicum* ATCC 13869 | 95. 6 | 50.0 | 0.0 | 0.0 | - |
| C. gl::argF* | 44. 2 | 50.0 | 13.3 | 26.6 | 13 |
| C. gl::argF*_PbetP-carA | 46. 8 | 50.0 | 15.9 | 31.8 | 16 |
| C. gl::argF*_carA(g1t) | 45. 3 | 50.0 | 15.3 | 30.6 | 15 |
| C. gl::argF*_carB(a1t) | 43. 9 | 50.0 | 15.4 | 30.8 | 15 |
| C. gl::argF*_argR* | 37. 9 | 50.0 | 17.2 | 34.4 | 17 |
| C. gl::argF*_argR*_PbetP-carA | 43. 6 | 50.0 | 20.9 | 41.8 | 21 |
| C. gl::argF* argR* carA(g1t) | 40. 5 | 50.0 | 20.1 | 40.2 | 20 |
| C. gl::argF* argR* carB(a1t) | 39. 8 | 50.0 | 20.5 | 41.0 | 21 |

As shown in Table 4, C. gl::argF*_PbetP-carA, C. gl::argF*_carA(g1t), and C. gl::argF*_carB(a1t) strains showed increased production ability and yield, as compared to the control strain C. gl::argF*. Similarly, C. gl::argF*_argR*_PbetP-carA, C. gl::argF*_argR*_carA(g1t), C. gl::argF*_argR*_carB(a1t) strains showed increased production ability and yield, as compared to the control strain C. gl::argF*_argR*.

Based on the above description, a person skilled in the art to which the present disclosure pertains can understand that the present disclosure may be embodied in other specific forms without departing from the technical spirit or essential characteristics thereof. In this regard, the embodiments described above should be understood to be illustrative rather than restrictive in every respect. The scope of the present disclosure should be construed as the meaning and scope of the appended claims rather than the detailed description, and all changes or variations derived from the equivalent concepts falling within the scope of the present disclosure.

## Claims

1. A microorganism of the genus *Corynebacterium* having L-amino acid-producing ability, wherein activity of carbamoyl phosphate synthetase is weakened.

2. The microorganism of claim 1, wherein the L-amino acid is any one or more selected from the group consisting of L-ornithine, L-glutamate, L-aspartate, and L-phenylalanine.

3. The microorganism of claim 1, wherein activity of any one or more selected from the group consisting of a carbamoyl phosphate synthetase small subunit (CarA) and a carbamoyl phosphate synthetase large subunit (CarB) is weakened.

4. The microorganism of claim 1, wherein the carbamoyl phosphate synthetase is encoded by carAB operon gene.

5. The microorganism of claim 3, wherein the CarA and CarB have amino acid sequences of SEQ ID NO: 1 and SEQ ID NO: 2 or amino acid sequences having 90% or more homology thereto, respectively.

6. The microorganism of claim 1, wherein activity of ornithine carbamoyltransferase subunit F (ArgF) is further weakened.

7. The microorganism of claim 1, wherein activity of arginine repressor (ArgR) is further weakened.

8. The microorganism of claim 1, wherein the microorganism of the genus *Corynebacterium* is *Corynebacterium glutamicum.*

9. A method of producing L-amino acids, the method comprising the step of culturing, in a medium, a microorganism of the genus *Corynebacterium* having L-amino acid-producing ability, wherein activity of carbamoyl phosphate synthetase is weakened.

10. The method of claim 9, wherein the L-amino acid is any one or more selected from the group consisting of L-ornithine, L-glutamate, L-aspartate, and L-phenylalanine.

11. The method of claim 9, wherein activity of ornithine carbamoyltransferase subunit F (ArgF) is further weakened in the microorganism.

12. The method of claim 9, wherein activity of arginine repressor (ArgR) is further weakened in the microorganism.

13. A composition for producing L-amino acids, the composition comprising a microorganism of the genus *Corynebacterium,* wherein activity of carbamoyl phosphate synthetase is weakened; a medium in which the microorganism is cultured; or a combination thereof.

14. Use of a microorganism of the genus *Corynebacterium,* wherein carbamoyl phosphate synthetase is weakened, in producing L-amino acids.
